# EUROPEAN PATENT APPLICATION

(11) **EP 4 696 687 A1**
(43) Date of publication of application: **18.02.2026**
(21) Application number: 24788137.8
(22) Date of filing: 11.04.2024
(51) Int. Cl.: C07D 405/06, C07D 307/33, A61K 31/415, A61P 27/02

(54) **INTERMEDIATE COMPOUNDS OF PILOCARPINE AND PREPARATION METHOD THEREFOR**

(30) Priority: 14.04.2023 CN 202310414024
(71) Applicant: Zhejiang Ausun Pharmaceutical Co., Ltd., Taizhou, Zhejiang 317016 (CN)
(72) Inventor: GUO, Jiajia, Zhejiang 317016 (CN); DAI, Chunguang, Zhejiang 317016 (CN); ZHANG, Lirong, Zhejiang 317016 (CN); YU, Guanneng, Zhejiang 317016 (CN); ZHANG, Shengkang, Zhejiang 317016 (CN); ZHENG, Zhiguo, Zhejiang 317016 (CN)
(74) Representative: Bianchetti & Minoja with Trevisan & Cuonzo IPS SRL
(86) International application number: PCT/CN2024/087193
(87) International publication number: WO 2024/213035

(57) **Abstract**

The present invention relates to intermediate compounds of pilocarpine and preparation methods therefor. In particular, the present invention relates to compounds of formula V and formula I for preparing pilocarpine and a preparation method therefor. In the method, a compound of formula II is used as a raw material, and a compound of formula V is obtained after halogenation hydrolysis, oxidation and substitution reactions; and then the compound of formula V is subjected to reduction reaction and ring-forming reaction to obtain the compound of formula I. The preparation method of the present invention has the advantages of mild reaction conditions, simple reaction process, high overall yield and a high purity etc., and is particularly suitable for industrial production.

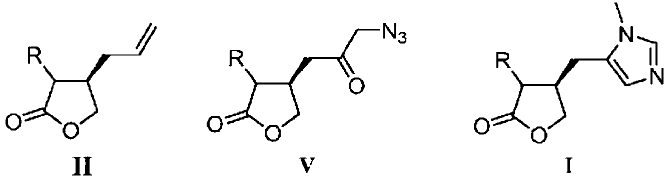

## Description

### TECHNICAL FIELD

The present disclosure relates to the field of medicinal chemistry, more particularly, the present disclosure relates to intermediate compounds for synthesizing pilocarpine and preparation methods for such intermediate compounds.

### BACKGROUND

Glaucoma is an eye disease in which the intraocular pressure increases intermittently or continuously. Persistent ocular hypertension can cause damage to various tissues of the eyeball and visual functions. The visual field can be completely lost, and blindness may occur if not treated in time.

Pilocarpine is an alkaloid extracted from the leaves of Pilocarpus plants *Pilocarpus jaborandi Holmes* and *Pilocarpus microphyllus Stapf,* which has the effect in simulating acetylcholine.

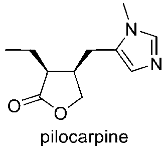

Pilocarpine is useful in the treatment of primary glaucoma, including open-angle and angle-closure glaucoma. Compared with physostigmine, pilocarpine has a mild and short-lived effect, and its aqueous solution is more stable. Pilocarpine can also be used for salivary gland hypofunction, and its oral tablet SALAGEN can relieve xerostomia. In addition, pilocarpine can also be used for miosis during cataract intraocular lens implantation surgery and symptomatic treatment of atropine drug poisoning. At present, medicinal pilocarpine is isolated and extracted from plants. However, with the increasing requirements for environmental protection, extraction from plants becomes more and more difficult. Moreover, in the case of obtaining pilocarpine by chemical synthesis, there are still many difficulties with the currently known methods.

From the literature Tetrahedron, 1972, 28, 967-972, patent application JP03161481 and US 5182198, it can be seen that the current methods for synthesizing pilocarpine have a long route and use dangerous or expensive materials such as metallic sodium and noble metal rhodium, and use enzymatic hydrolysis resolution twice. However, the enzyme is expensive, the amount used is large, and the yield of the resolution is low, all of these bring great inconvenience to industrial production and significantly increasing cost for industrial production.

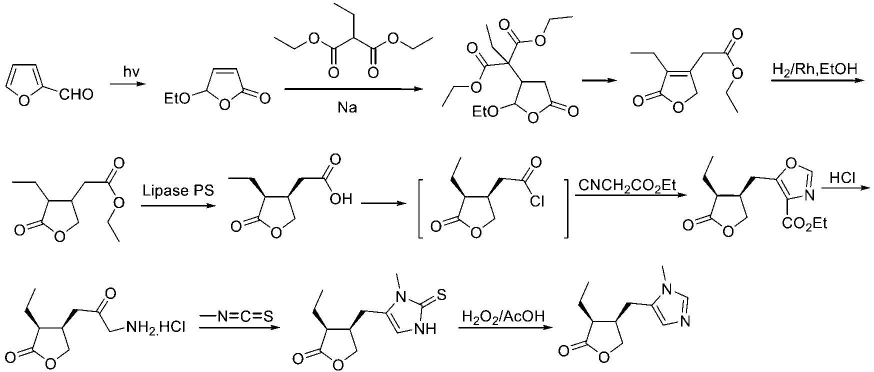

Therefore, how to prepare pilocarpine simply and efficiently has become the key to solving the problem of industrial production of pilocarpine.

### CONTENT OF THE INVENTION

The purpose of the present disclosure is to overcome the deficiencies of the prior art, and to provide a preparation method of a novel pilocarpine intermediate, namely compound of formula I, and its use in preparing pilocarpine. The method has the advantages of mild reaction conditions, simple reaction process, convenient operations, high overall yield of the target product and up to 99.9% of purity of the target product, and is particularly suitable for industrial production.

In the first aspect, the present disclosure provides a method for preparing a compound of formula V, comprising the following steps:
step 1: reacting a compound of formula II with a halogenating reagent, and then hydrolyzing to obtain a compound of formula III;
step 2: reacting the compound of formula III with an oxidizing agent to obtain a compound of formula IV;
step 3: reacting the compound of formula IV with an azidation reagent to obtain a compound of formula V; 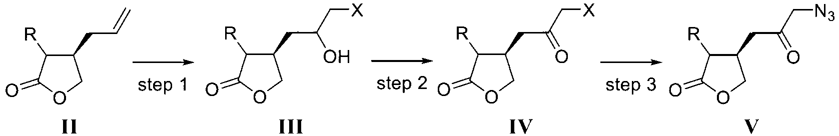
wherein, R is H or ethyl; X is halogen selected from Cl, Br or I.

In an embodiment, the halogenating reagent in step 1 is selected from one of chlorine, NCS, trichloroisocyanuric acid, 1,3-dichloro-5,5-dimethylhydantoin, lithium chloride, sodium chloride, potassium chloride, tetrabutylammonium chloride, bromine, NBS, 1,3-dibromo-5,5-dimethylhydantoin, tribromoisocyanuric acid, lithium bromide, sodium bromide, potassium bromide, tetrabutylammonium bromide, iodine, 1,3-diiodo-5,5-dimethylhydantoin, lithium iodide, sodium iodide, potassium iodide and tetrabutylammonium iodide, or a combination of two or more of the above halogenating reagents.

In an embodiment, the reaction solvent in step 1 is selected from one of toluene, tetrahydrofuran, 2-methyltetrahydrofuran, acetonitrile, dichloromethane, chloroform, methyl tert-butyl ether, ethyl acetate, hexane, n-heptane, acetone, or a combination of two or more of the above reaction solvents.

In an embodiment, the oxidizing agent in step 2 is selected from one of Dess-Martin periodinane, hydrogen peroxide, Jones reagent, PCC, PDC and Swern reagent, or a combination of two or more of the above oxidizing agents.

In an embodiment, the azidation reagent in step 3 is selected from one of sodium azide, azidotrimethylsilane, diphenylphosphoryl azide, tributyltin azide, tetrabutylammonium azide, tetramethylguanidinium azide and ethyl azidoacetate, or a combination of two or more of the above azidation reagents.

In an embodiment, the reaction solvent in step 2 and step 3 is selected from one of toluene, tetrahydrofuran, 2-methyltetrahydrofuran, acetonitrile, dichloromethane, chloroform, methyl tert-butyl ether, ethyl acetate, hexane, n-heptane, acetone, or a combination of two or more of the above reaction solvents.

In the second aspect, the present disclosure provides a method for preparing a compound of formula I, comprising the following steps:
step 4: subjecting the compound of formula V and a reducing agent to a reduction reaction, to obtain a compound of formula VI;
step 5: reacting the compound of formula VI with methyl isothiocyanate to obtain a compound of formula VII or a salt thereof;
step 6: subjecting the compound of formula VII to an oxidative desulfurization, to obtain a compound of formula I; 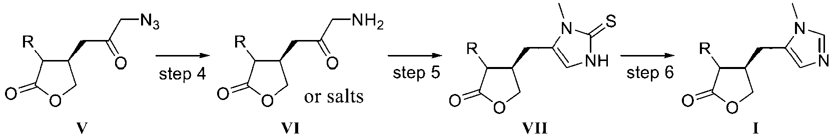
wherein, R is H or ethyl.

In an embodiment, the reduction reaction in step 4 is a catalytic hydrogenation reduction reaction or a reduction reaction using a reducing agent, wherein a combination of a catalyst and a reducing agent is used in the catalytic hydrogenation reduction reaction, the catalyst is selected from Ni, Pd/C, Pt/C, PtO₂, palladium on barium sulfate or any combination thereof, and the reducing agent is selected from hydrogen, ammonium chloride, formic acid and ammonium formate or any combination thereof; the reducing agent used in the reduction reaction using a reducing agent is selected from one of sodium borohydride, lithium borohydride, sodium cyanoborohydride, potassium borohydride, borane, Red-Al and lithium aluminum hydride, or a combination of two or more of the above reducing agents.

In an embodiment, the salt in step 5 is selected from hydrochloride, hydrobromide, hydroiodide, sulfate, phosphate or nitrate.

In an embodiment, the oxidizing agent in step 6 is selected from hydrogen peroxide, Jones reagent or sodium nitrite.

In an embodiment, the reaction solvent in step 4, step 5 and step 6 is selected from one of methanol, ethanol, isopropanol, toluene, tetrahydrofuran, 2-methyltetrahydrofuran, acetonitrile, dichloromethane, chloroform, methyl tert-butyl ether, ethyl acetate, hexane, n-heptane and acetone, or a combination of two or more thereof.

In the third aspect, the present disclosure provides a method for preparing pilocarpine or a hydrochloride or nitrate thereof.

For the compound of formula I wherein R is ethyl, which is referred to as the compound of formula I', the method comprises a step of salifying and crystallizing the compound of formula I' to obtain a hydrochloride or nitrate of pilocarpine:

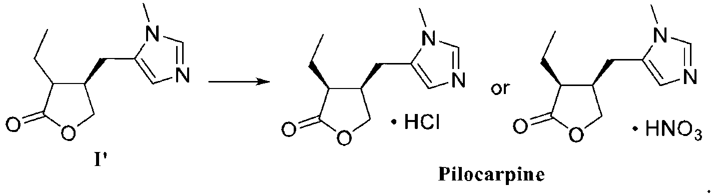

In an embodiment, the compound of formula I' is dissolved in an alcohol reagent, and hydrochloric acid or nitric acid is added for recrystallization to obtain a hydrochloride or nitrate pilocarpine.

Said alcohol solvent is selected from, for example, methanol, ethanol, n-propanol, isopropanol, n-butanol, or any combination thereof.

For the compound of formula I wherein R is H, which is referred to as the compound of formula I", the method comprises a step of reacting the compound of formula I" with a halogenating reagent CH₃CH₂X to obtain a compound of formula I', in which R is ethyl, followed by salifying and crystallizing the compound of formula I' to obtain hydrochloride or nitrate of pilocarpine:

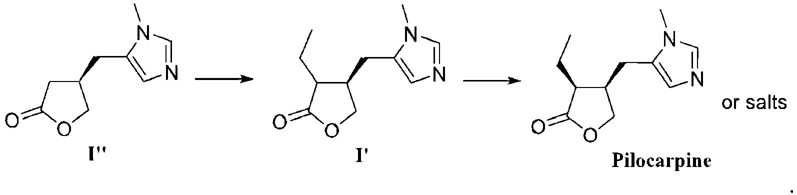

In a specific embodiment, the compound of formula I" is dissolved in an organic solvent, and a strong base is added at -10 to 5 °C. After the addition is completed, the mixture is stirred for 0.5-1.5 hours while keeping at this temperature. Then, ethyl bromide is added dropwise, and then the reaction is heated to 15 to 25 °C and stirred for 2.0-3.0 hours. The reaction is quench by adding a solution of hydrochloric acid or ammonium chloride, washed, dried over anhydrous sodium sulfate, filtered, and concentrated to dryness, to obtain the compound of formula I'.

The organic solvent is selected from, for example, toluene, acetonitrile, isopropyl acetate, diethyl ether, isopropyl ether, methyl tert-butyl ether, tetrahydrofuran, methyltetrahydrofuran, and 1,4-dioxane.

The strong base is selected from, for example, lithium hexamethyldisilazide, sodium hexamethyldisilazide or LDA.

The methods described in the first, second and third aspects of the present application can be combined arbitrarily as needed. For example, the present disclosure relates to the following method for preparing the compound of formula I, comprising the following steps:
step 1: reacting the compound of formula II with a halogenating reagent, then hydrolyzing to obtain the compound of formula III;
step 2: reacting the compound of formula III with an oxidizing agent to obtain the compound of formula IV;
step 3: reacting the compound of formula IV with an azidation reagent to obtain the compound of formula V;
step 4: subjecting the compound of formula V and a reducing agent to a reduction reaction, to obtain the compound of formula VI;
step 5: reacting the compound of formula VI with methyl isothiocyanate to obtain the compound of formula VII or the salt thereof;
step 6: subjecting the compound of formula VII to an oxidative desulfurization, to obtain the compound of formula I; 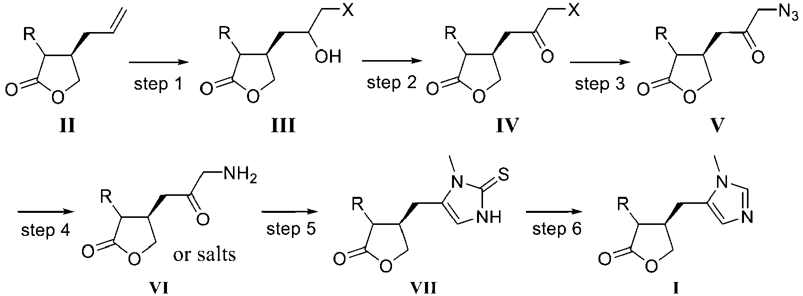
wherein R is H or ethyl; X is halogen selected from Cl, Br or I;
the reaction conditions of each step are as described above.

In a preferred embodiment, the above method further comprises a step of preparing the hydrochloride or nitrate of pilocarpine as described in the third aspect of the present application.

In the fourth aspect, the present disclosure provides the compound of formula V or a salt thereof: 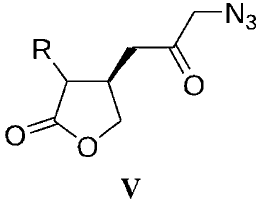 wherein, R is H or ethyl.

The present disclosure also provides the compound of formula IV or a salt thereof: 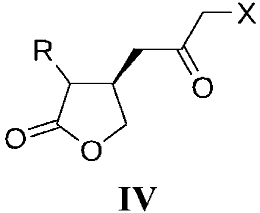
wherein, R is H or ethyl, and X is halogen selected from Cl, Br or I;
with the proviso that the following compounds of formula IV wherein R is H, and X is Cl or Br; or wherein R is ethyl, and X is Cl are excluded:

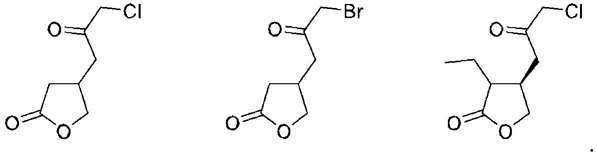

The present disclosure also provides the following compounds:

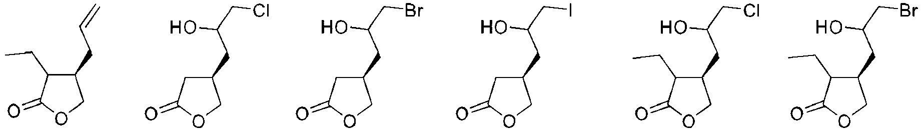

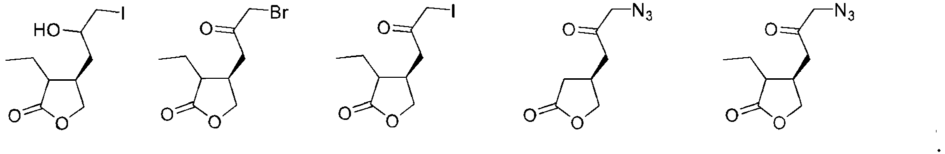

### BRIEF DESCRIPTION OF THE DRAWINGS

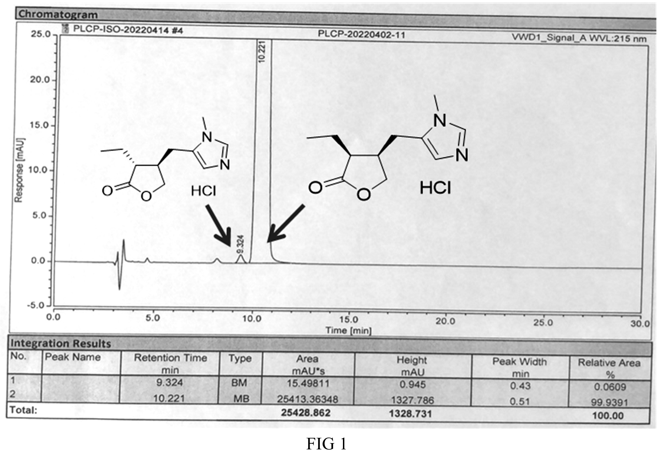

FIG 1 shows the HPLC test results of pilocarpine hydrochloride obtained in Example 8.

### SPECIFIC MODE FOR CARRYING OUT THE INVENTION

Abbreviations used herein and other description:
NBS refers to N-bromosuccinimide.
PCC refers to pyridinium chlorochromate.
PDC refers to pyridinium dichromate.
DMSO refers to dimethyl sulfoxide.
DMF refers to N,N-dimethylformamide.
LDA refers to lithium diisopropylamide.

The used organic solvent is not particularly limited, as long as it can dissolve the starting material compound. Preferably, the used organic solvent may be selected from one or more of toluene, hexane, n-heptane, methyl acetate, ethyl acetate, isopropyl acetate, acetonitrile, tetrahydrofuran, isopropyl ether, methyl tert-butyl ether, dichloromethane, acetone, methanol, ethanol, and isopropanol.

In the above method, the reaction temperature is not particular limited, and belongs to a conventional reaction condition.

In the above method, conventional quenching of reaction refers to adding an aqueous solution, an ammonium chloride solution, an aqueous hydrochloric acid solution, an aqueous phosphoric acid solution, and the like to the reaction solution to stop the reaction or disrupt the reaction system.

In the above method, the conventional extracting process refers to a process of dissolving the reaction product in an organic solvent or dissolving the reaction product in an aqueous phase to separate them from other substances in the reaction system or to remove impurities.

The compound of formula II is prepared by referring to the method described in Org.Biomol.Chem., 2017, 15, 3728-3735, CN108929289 and Organic Letters, 2011, 13, 1, 118-121 or a similar method.

The method of the present disclosure will be further illustrated by the following examples. It should be understood that the following examples are provided only for the purpose of enabling a better understanding of the present invention, and are not intended to limit the scope of the present invention in any way.

Unless otherwise specified, the raw materials and reagents used in the examples of the present invention were commercially available or obtained by known methods, purity and chiral values were measured by high performance liquid chromatography, and identification of the target product was confirmed by consistency of HPLC retention values of the standards.

### Example 1: Preparation of the compound of formula III (R is ethyl)

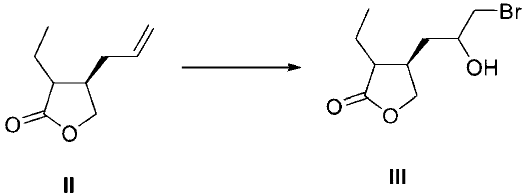

39g of compound II, 53.5g of NBS, 450mL of acetonitrile and 150mL of water were added to a four-necked flask and stirred at 25°C for 12 hours. After TLC monitored the completion of the reaction, the reaction solution was concentrated under reduced pressure. The concentrate was added 100 mL of water, and extracted with 3×100mL of methyl tert-butyl ether. The organic layers were combined, washed with water once, and evaporated to dryness to obtain 45.0g of oil, with a yield of 70.8%, and directly used in the next step without purification.

### Example 2: Preparation of the compound of formula IV (R is ethyl)

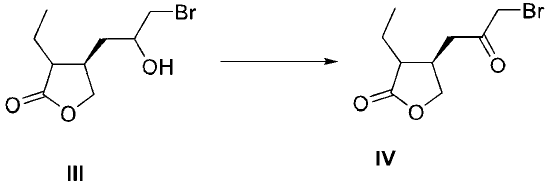

27.0 g of compound III, 58.4 g of Dess-Martin periodinane and 350 mL of dichloromethane were added to a four-necked flask and stirred at 25 °C for 8 hours. After TLC monitored the completion of the reaction, the reaction was quenched by adding 200 mL of saturated NaHSO₃ solution. The mixture was extracted, and the layers were separated. The organic layer was washed with NaHCO₃ solution twice and with brine once. After evaporation to dryness, 23.6 g of pale yellow oil was obtained by column chromatography with a yield of 88.0%.
**¹H NMR** (400 MHz, CDCl₃) δ 4.53 (dd, *J =* 9.3, 7.5 Hz, 1H), 3.87 (s, 2H), 3.79 (dd, *J* = 9.3*,* 7.5 Hz, 1H), 3.10 - 3.02 (m, 1H), 2.86 - 2.77 (m, 1H), 2.75 - 2.65 (m, 1H), 2.22 - 2.15 (m, 1H), 1.74 - 1.68 (m, 2H), 1.01 (t, *J =* 6.3 Hz, 3H).

### Example 3: Preparation of the compound of formula V (R is ethyl)

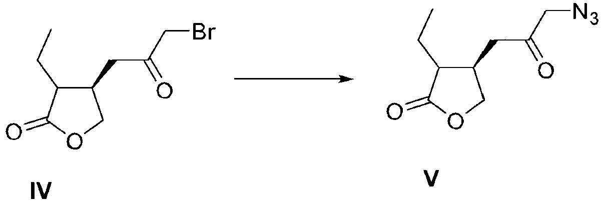

Under the protection of nitrogen, 21.6g of compound IV and 200mL of acetone were added to a four-necked flask, and 8.4g of NaN₃ solid was added in batches. The mixture was stirred at 25°C for 13 hours. After TLC monitored the completion of the reaction, 200mL of water and 200mL of ethyl acetate were added. The mixture was extracted, and the layers were separated. The organic layer was washed with water for 3 times, and evaporated to dryness to obtain 16.7g of oil, with a yield of 91.5%, which is directly used in the next step without purification.
**¹H NMR** (400 MHz, CDCl₃) δ 4.47 (dd, J = 9.3, 7.5 Hz, 1H), 3.93 (s, 2H), 3.72 (dd, J = 9.4, 7.5 Hz, 1H), 2.80 (dd, J = 17.7, 4.1 Hz, 1H), 2.68 - 2.50 (m, 2H), 2.12 (dt, J = 8.5, 6.1 Hz, 1H), 1.70 - 1.50 (m, 2H), 0.93 (t, *J =* 6.4 Hz, 3H).

### Example 4: Preparation of the compound of formula VI (R is ethyl)

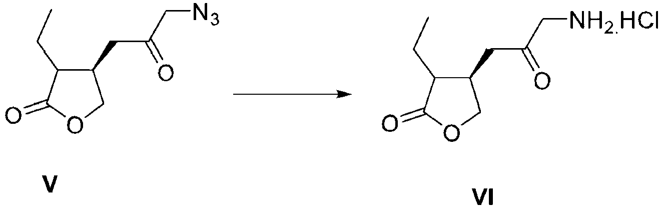

A hydrogenation reactor was charged with 14.7 g of compound V, 12 mL of concentrated hydrochloric acid, 150 mL of methanol and 1.5 g of 10% palladium/carbon, and was purged with hydrogen for three times. The reaction was stirred at 25° C for 6 hours under 1 kg of hydrogen pressure. After TLC monitored the completion of the reaction, and the reaction was filtered and evaporated to dryness to obtain 15.2 g of crude product with a yield of 99.0%, which is directly used in the next step without purification.

### Example 5: Preparation of the compound of formula VII (R is ethyl)

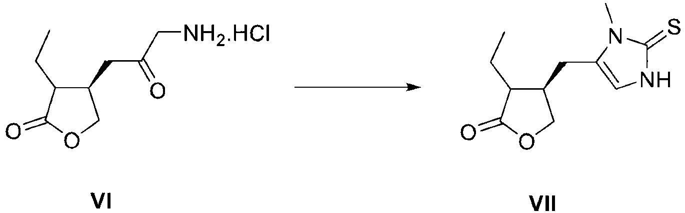

15.0 g of compound VI, 9.8 g of potassium carbonate, 16.3 g of methyl isothiocyanate and 150 mL of 70% tetrahydrofuran (105 mL of tetrahydrofuran and 45 mL of water) were added to a four-necked flask, and stirred at 25°C for 14 hours. After TLC monitored the completion of the reaction, the reaction was extracted with 5×80 mL of dichloromethane. The organic layers were combined and evaporated to dryness to obtain 10.7 g of solid, with a yield of 65.9%, which is directly used in the next step without purification.

### Example 6: Preparation of the compound of formula I (R is ethyl)

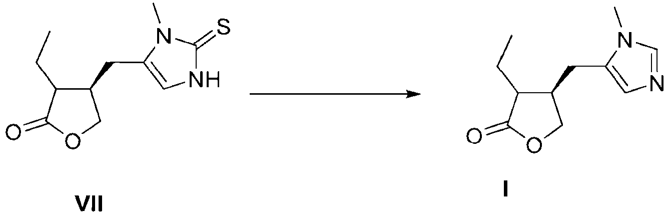

10.0g of compound VII, 180mL of water and 20mL of nitric acid were to a four-necked flask, and 4.0g of solid sodium nitrite was added under an ice-water bath. After keeping at this temperature for 0.5 hours, the reaction was warmed to 25°C for 3 hours. After TLC monitored the completion of the reaction, the impurities were extract with 2×80ml of dichloromethane and the organic layer was discarded. The aqueous layer was adjusted to pH 7-8 with aqueous ammonia, extracted with 4×100mL of dichloromethane. The dichloromethane layers were combined, evaporated to dryness, and 7.49g of liquid was obtained by column chromatography with a yield of 86.4%, e.e.=27.2%.

**¹H NMR** (500 MHz, d-DMSO) δ 9.12 (s, 1H), 7.59 (s, 1H), 4.41 (t, *J =* 8.2 Hz, 1H), 3.90 (t, *J =* 8.4 Hz, 1H), 3.80 (s, 3H), 2.99 (dd, *J =* 15.8, 5.1 Hz, 1H), 2.85 (dd, *J* = 15.9, 9.5 Hz, 1H), 2.74 - 2.63 (m, 1H), 2.46 (dt, *J =* 8.9, 6.0 Hz, 1H), 1.66 - 1.57 (m, 2H), 0.93 (t, *J =* 7.4 Hz, 3H).

### Example 7: Preparation of the compound of formula I (R is ethyl)

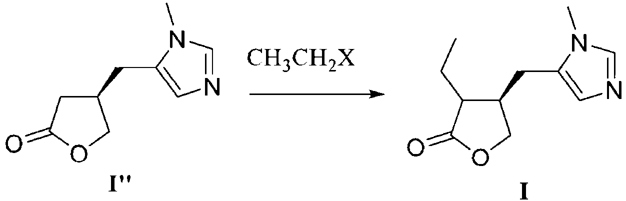

1.8 g of compound I' was dissolved in 100 mL of anhydrous tetrahydrofuran, and 6 mL of LDA (2.0 M) was added dropwise in an ice-water bath. After the addition was completed, the reaction was stirred at this temperature for 0.5 hours, then 1.5 g of ethyl bromide was added dropwise. After the addition was completed, the reaction was slowly warmed to room temperature and stirred for 2-3 hours. After TLC monitored the completion of the reaction, the reaction was quenched by adding saturated ammonium chloride solution in an ice bath, 30 ml of ethyl acetate was added. The impurities were extracted twice. The organic phases were combined, washed with saturated brine, evaporated to dryness, and 1.5 g of liquid was obtained by column chromatography with a yield of 79.4% and e.e.=25.6%.

### Example 8: Preparation of hydrochloride compound (R is ethyl)

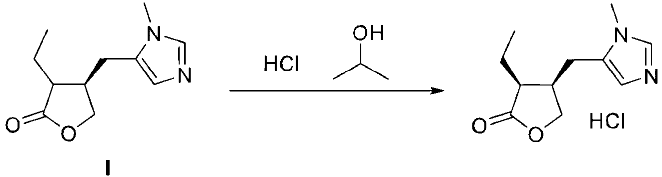

To a four-necked flask was added 6.0 g of compound I (e.e. = 27.2%), 50 mL of isopropanol and 5 mL of concentrated hydrochloric acid were added. The reaction was warmed to 45-50 ° C and stirred for 1-2 hours at this temperature. The reaction was concentrated under reduced pressure to obtain crude pilocarpine hydrochloride. Then, 30 mL of isopropanol was added, the mixture was warmed to 65-75 ° C and stirred for 0.5-1.0 hours at this temperature. Then, the mixture was slowly cooled to room temperature, filtered, and the filter cake was washed with 10 mL of isopropanol and dried to obtain 3.0 g of white pilocarpine hydrochloride.

The above 3.0 g product was added into 30 mL of isopropanol for a second recrystallization and dried to obtain 2.6 g of pilocarpine hydrochloride with a total yield of 43.1% and e.e.=99.9%, as shown in FIG 1.

¹H NMR (500 MHz, DMSO) δ 9.21 (d, J = 0.9 Hz, 1H), 7.62 (d, J = 0.9 Hz, 1H), 4.27 (dd, J = 9.1, 5.8 Hz, 1H), 3.98 (dt, J = 14.2, 7.1 Hz, 1H), 3.82 (s, 3H), 3.07 - 2.94 (m, 1H), 2.80 (dd, J = 15.4, 7.0 Hz, 2H), 2.56 (dd, J = 16.0, 11.2 Hz, 1H), 1.68 (dp, J = 14.7, 7.4 Hz, 1H), 1.58 - 1.47 (m, 1H), 1.01 (t, J = 7.4 Hz, 3H).

The specific embodiments and examples described above further illustrate the purpose, technical solutions and beneficial effects of the present invention in detail. It should be understood that the specific embodiments and examples described above are only exemplary illustrations of the present invention and are not intended to limit the scope of the disclosure of this application. Any modifications, equivalents, improvements and the like made within the spirit and principles of the present invention shall be included in the protection scope of the disclosure of this application.

## Claims

1. A method for preparing a compound of formula V, comprising the following steps:
step 1: reacting a compound of formula II with a halogenating reagent, and then hydrolyzing to obtain a compound of formula III;
step 2: reacting the compound of formula III with an oxidizing agent to obtain a compound of formula IV; and
step 3: reacting the compound of formula IV with an azidation reagent to obtain a compound of formula V; 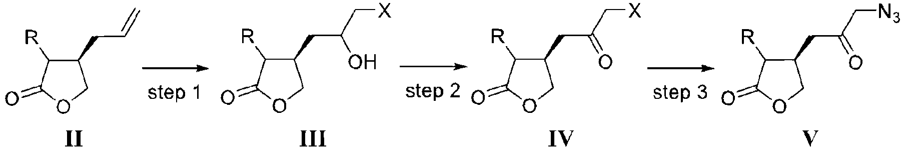
wherein, R is H or ethyl; X is halogen selected from Cl, Br or I.

2. The method according to claim 1, wherein the halogenating reagent in step 1 is selected from one of chlorine, NCS, trichloroisocyanuric acid, 1,3-dichloro-5,5-dimethylhydantoin, lithium chloride, tetrabutylammonium chloride, bromine, NBS, 1,3-dibromo-5,5-dimethylhydantoin, tribromoisocyanuric acid, lithium bromide, tetrabutylammonium bromide, iodine, 1,3-diiodo-5,5-dimethylhydantoin, lithium iodide, sodium iodide, potassium iodide and tetrabutylammonium iodide, or a combination of two or more of the above halogenating reagents.

3. The method according to claim 1, wherein the oxidizing agent in step 2 is selected from one of Dess-Martin periodinane, hydrogen peroxide, Jones reagent, PCC, PDC and Swern reagent, or a combination of two or more of the above oxidizing agents.

4. The method according to claim 1, wherein the azidation reagent in step 3 is selected from one of sodium azide, azidotrimethylsilane, diphenylphosphoryl azide, tributyltin azide, tetrabutylammonium azide, tetramethylguanidinium azide and ethyl azidoacetate, or a combination of two or more of the above azidation reagents.

5. A method for preparing a compound of formula I comprising the following steps:
step 4: subjecting a compound of formula V and a reducing agent to a reduction reaction, to obtain a compound of formula VI;
step 5: reacting the compound of formula VI with methyl isothiocyanate to obtain a compound of formula VII or a salt thereof;
step 6: subjecting the compound of formula VII to an oxidative desulfurization, to obtain a compound of formula I; 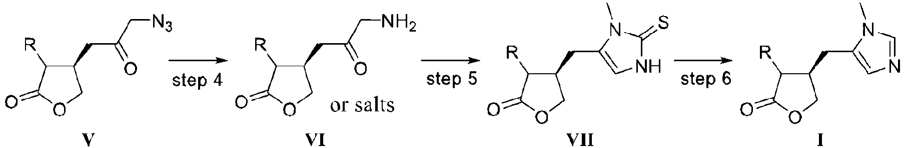
wherein, R is H or ethyl.

6. The method according to claim 5, wherein the reduction reaction in step 4 is a catalytic hydrogenation reduction reaction or a reduction reaction using a reducing agent, wherein a combination of a catalyst and a reducing agent is used in the catalytic hydrogenation reduction reaction, the catalyst is selected from Ni, Pd/C, Pt/C, PtO₂, palladium on barium sulfate or any combination thereof, and the reducing agent is selected from hydrogen, ammonium chloride, formic acid, ammonium formate or any combination thereof; the reducing agent used in the reduction reaction using a reducing agent is selected from one of sodium borohydride, lithium borohydride, sodium cyanoborohydride, potassium borohydride, borane, Red-Al and lithium aluminum hydride, or a combination of two or more of the above reducing agents.

7. The method according to claim 5, wherein the salt in step 5 is selected from hydrochloride, hydrobromide, hydroiodide, sulfate, phosphate or nitrate.

8. The method according to claim 5, wherein the oxidizing agent in step 6 is selected from hydrogen peroxide, Jones reagent or sodium nitrite.

9. The method according to claim 5, when R is ethyl, the compound is referred to as compound of formula I', and the method further comprises a step of salifying and crystallizing the compound of formula I' to obtain a hydrochloride or nitrate of pilocarpine:

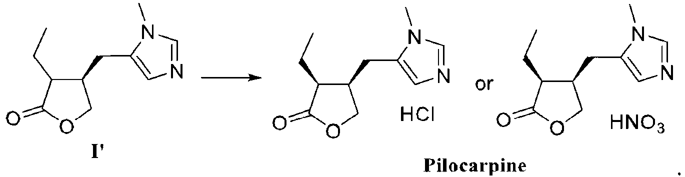

10. The method according to claim 5, when R is H, the compound is referred to as formula I", and the method further comprises a step of reacting the compound of formula I" with a halogenating reagent CH₃CH₂X, followed by salifying and crystallizing, to obtain hydrochloride or nitrate of pilocarpine: 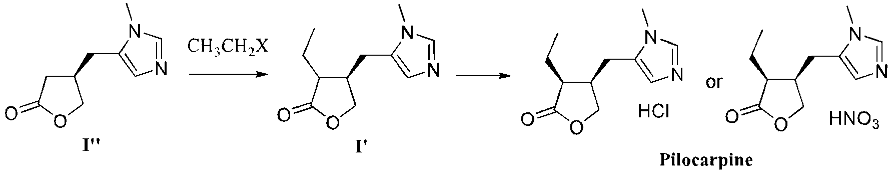 wherein, X is halogen selected from Cl, Br or I.

11. The method according to claim 9 or 10, wherein the salifying and crystallizing is carried out in an alcohol solvent, for example, one of methanol, ethanol, n-propanol, isopropanol, n-butanol, or any combination thereof, using hydrochloric acid or nitric acid.

12. The method according to any one of claims 5 to 11, comprising the following steps:
step 1: reacting a compound of formula II with a halogenating reagent, then hydrolyzing to obtain a compound of formula III;
step 2: reacting the compound of formula III with an oxidizing agent to obtain a compound of formula IV;
step 3: reacting the compound of formula IV with an azidation reagent to obtain the compound of formula V;
step 4: subjecting the compound of formula V and a reducing agent to a reduction reaction, to obtain the compound of formula VI;
step 5: reacting the compound of formula VI with methyl isothiocyanate to obtain the compound of formula VII or the salt thereof;
step 6: subjecting the compound of formula VII to an oxidative desulfurization, to obtain the compound of formula I; 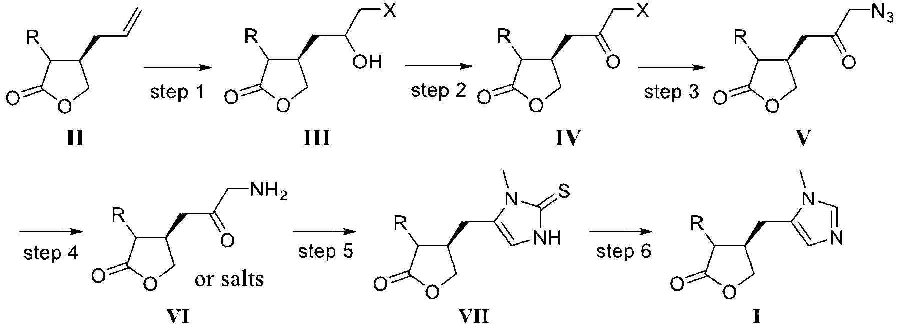
wherein R is H or ethyl; X is halogen selected from Cl, Br or I;
the reaction conditions of steps 1-3 are as described in any one of claims 1-4.

13. A compound of formula V or a salt thereof: 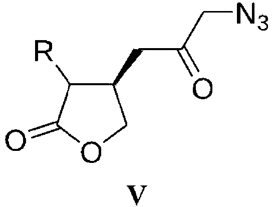 wherein, R is H or ethyl.

14. A compound of formula IV or a salt thereof: 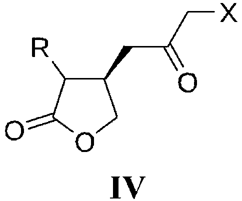
wherein, R is H or ethyl, X is halogen selected from Cl, Br or I,
with the proviso that the compounds of formula IV wherein R is H, and X is Cl or Br; or wherein R is ethyl, and X is Cl are excluded.

15. A compound or a salt thereof, wherein the compound is selected from:
